# EUROPEAN PATENT SPECIFICATION

(11) **EP 2 563 464 B1**
(45) Date of publication and mention of the grant of the patent: **06.06.2018**
(21) Application number: 11720231.7
(22) Date of filing: 29.04.2011
(51) Int. Cl.: A61N 1/375, H01L 21/56, A61N 1/36, A61B 5/04, A61N 1/05, H01L 23/00

(54) **IMPROVED BIOCOMPATIBLE BONDING METHOD**
VERBESSERTES BIOKOMPATIBLES BINDUNGSVERFAHREN
PROCÉDÉ AMÉLIORÉ DE LIAISON BIOCOMPATIBLE

(30) Priority: 30.04.2010 US 330089 P; 30.04.2010 US 330204 P
(43) Date of publication of application: 06.03.2013
(73) Proprietor: Second Sight Medical Products, Inc., Sylmar, CA 91342 (US)
(72) Inventor: FARAJI, Boozarjomehr, Valencia, CA 91354 (US); ZHOU, David, Daomin, Saugus, CA 91390 (US); GREENBERG, Robert, J., Los Angeles, CA 90068 (US); OK, Jerry, Canyon Country, CA 91350 (US); TALBOT, Neil, Hamilton, La Crescenta, CA 91214 (US); LITTLE, James, Singleton, Saugus, CA 91350 (US)
(74) Representative: Graham, Emma Louise
(86) International application number: PCT/US2011/034471
(87) International publication number: WO 2011/137298

(56) References cited:
- US-A1- 2003 032 275
- US-A1- 2003 192 784
- US-A1- 2007 207 569
- US-A1- 2008 182 400
- US-A1- 2010 052 163

## Description

### BACKGROUND OF THE INVENTION

### Field of the Invention

This invention relates to an improved method for attaching a flexible circuit, such as attaching an electrode array to an implantable hermetic package, as for packaging electronics.

### Description of Related Art

US 2007/0207569 A1 discloses a biocompatible bonding method and electronics package suitable for implantation.

This application refers to US Patent No. 7,142,919 "Biocompatible Bonding Method and Electronics Package Suitable for Implantation" and US Patent No. 6,974,533 "Platinum Electrode and Method for Manufacturing the Same."

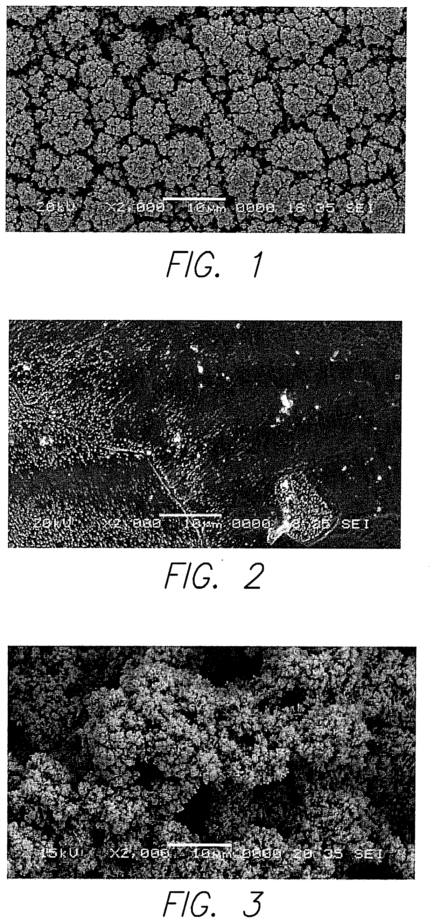

Greenberg, et at US Pat Pub. No. US 2008/0046021 teach a hermetic package for implantation in the human body having electrically conductive vias through the substrate and a flip chip bonded circuit or a wire bonded circuit in communication with the vias where a cover is bonded to the substrate such that the cover, substrate and vias form a hermetic package, as presented in **FIGS. 1, 2,** and **3****.**

Greenberg, et al. US Pat. Pub. No. 2009/0270958 teach an implantable hermetic electrode array for neural stimulation suitable to attach to neural tissue for a retinal electrode array for a visual prosthesis as presented in **FIGS. 1, 2,** and 3. Greenberg, et al. US Pat. No. 7,142,909 teach a method of bonding and an implantable electronics package for a flexible circuit such as for a retinal or cortical electrode array to enable restoration of sight to non-sighted individuals. The hermetically sealed electronics package is directly bonded to the flex circuit or electrode by electroplating a biocompatible material, such a platinum or gold to bond the flex circuit to the electronics package for biocompatible implantation in living tissue.

A microelectrode system used in neurostimulation and neurosensing typically includes an array of microelectrodes used as signal sources or a sensor interface for generating or receiving electrical signals, thereby to stimulate or sense activities in tissues. Schulman, et al. US Pat. No. 6,498,043, Schulman, et al. US Pat. No. 7,079,881 teach the use of ion beam assisted deposition (IBAD) to place metallization layers and an insulator on the substrate surface. Whalen, et al. US Pat. Pub. No. US 2007/0123766, teach the microelectrodes in a neurostimulation or sensing device are typically connected to an electronic device, for example, a microchip, by interconnects. The electronic device is preferably be protected in a fluid impermeable hermetic package and the interconnects are the only part of the device that penetrate through the fluid impermeable package. In the development of a microelectrode array embedded in a substrate, the substrate/electrode structure preferably resists fluid penetration so as to ensure the electronic device is not damaged by short circuiting or corrosion. Fluid penetration through the electrode or substrate structure can occur in one of the following ways: 1) through the electrode itself; 2) through the substrate; or 3) along the interface between the electrode and substrate. Appropriate material selection for the electrode and the substrate and appropriate manufacturing process are needed to produce a fluid impermeable microelectrode system.

The following US Patents relate to electronics packaging and platinum gray.
US Patent 7,904,148 Biocompatible Bonding Method and Electronics Package Suitable for Implantation
US Patent 7,887,681 Platinum Electrode Surface Coating and Method for Manufacturing the Same
US Patent 7,881,799 Retinal Prosthesis And Method of Manufacturing a Retinal Prosthesis
US Patent 7,873,419 Retinal Prosthesis And Method of Manufacturing a Retinal Prosthesis
US Patent 7,846,285 Biocompatible Electroplated Interconnection Bonding Method and Electronics Package Suitable for Implantation
US Patent 7,835,798 Electronics Package Suitable For Implantation
US Patent 7,813,796 Biocompatible Bonding Method and Electronics Package Suitable for Implantation
US Patent 7,725,191 Package For An Implantable Device
US Patent 7,666,523 Electrode Surface Coating and Method for Manufacturing the Same
US Patent 7,645,262 Biocompatible Bonding Method and Electronics Package Suitable for Implantation
US Patent 7,565,203 Package for an Implantable Medical Device
US Patent 7,480,988 Method and Apparatus for Providing Hermetic Electrical Feedthrough
US Patent 7,257,446 Package for an Implantable Medical Device
US Patent 7,211,103 Biocompatible Bonding Method and Electronics Package Suitable for Implantation
US Patent 7,142,909 Biocompatible Bonding Method and Electronics Package Suitable for Implantation
US Patent 6,974,533 Platinum Electrode and Method for Manufacturing the Same

### GLOSSARY

Terms are to be interpreted within the context of the specification and claims. The following terms of art are defined and shall be interpreted by these definitions. Medical terms that are not defined here shall be defined according to The American Heritage Stedman's Medical Dictionary, Houghton Mifflin, 1995. Terms that are not defined here shall be defined according to definitions from the ASM Metals Reference Book, 3rd Edition, 1993. Biocompatible. The ability of a long-term implantable medical device to perform its intended function, with the desired degree of incorporation in the host, without eliciting any undesirable local or systemic effects in that host. Regulatory agencies require that implanted objects or devices within the human body be biocompatible.

Body. The entire material or physical structure of an organism, especially of a human.

Bond. In welding, brazing, or soldering, the junction of joined parts. Where filler metal is used, it is the junction of the fused metal and the heat-affected base metal.

Braze. Bonding by heating an assembly to suitable temperature and by using a filler metal having a liquidus above 450°C (840°F) and below the solidus of the base metal. The filler metal is distributed between the closely fitted faying surfaces of the joint by capillary action.

Butt joint. A joint between two abutting members lying approximately in the same plane.

Cavity. The hollow area within the body, such as a sinus cavity, vagina, mouth, anus, or ear.

Filler metal. Metal added in making a brazed, soldered, or welded joint.

Hermetic. Completely sealed by fusion, soldering, brazing, etc., especially against the escape or entry of air, water, or other fluid.

Implant. To embed an object or a device in a body surgically along a surgically created implantation path.

Insert. To place an object or a device into a body cavity.

Joined. Fastened together by brazing, welding, or soldering.

Microstimulator. An implantable, biocompatible device having dimensions that are less than about 6 mm diameter and 60 mm in length that is capable of sensing or stimulating electrical signals within living tissue.

Silicone. Any of a group of non-hermetic, semi-inorganic polymers based on the structural unit R₂SiO, where R is an organic group, characterized by physiological inertness and used in adhesives, lubricants, protective coatings, electrical insulation, synthetic rubber, and prosthetic replacements for body parts.

Soldering. A group of processes that join metals by heating them to a suitable temperature below the solidus of the base metals and applying a filler metal having a liquidus not exceeding 450°C (840°F). Molten filler metal is distributed between the closely fitted surfaces of the joint by capillary action.

Solid-state welding. A group of processes that join metals at temperatures essentially below the melting points of the base materials, without the addition of a brazing or soldering filler metal. Pressure may or may not be applied to the joint.

Subcutaneous. Located, found, or placed just beneath the skin.

Surgery. A procedure involving the cutting or intrusive penetration of body tissue by cutting or penetration and not by inserting an object or a device into a naturally existing body cavity.

Surgical. Of, relating to, or characteristic of surgeons or surgery.

### SUMMARY OF THE INVENTION

The present invention is defined by the appended claims. The examples, embodiments, or aspects of the present description that do not fall within the scope of said claims are merely provided for illustrative purposes and do not form part of the invention.

### BRIEF DESCRIPTION OF THE SEVERAL VIEWS OF THE DRAWINGS

**FIG. 1** presents a platinum gray surface photomicrograph magnified 2000 times.
**FIG. 2** presents a shiny platinum surface photomicrograph magnified 2000 times.
**FIG. 3** presents a platinum black surface photomicrograph magnified 2000 times.
**FIG. 4** presents color density (D) values and lightness (l*) values for several representative samples of platinum gray, platinum black and shiny platinum.
**FIG. 5** presents a three-electrode electroplating cell with a magnetic stirrer.
**FIG. 6** presents a three-electrode electroplating cell in an ultrasonic tank.
**FIG. 7** presents a three-electrode electroplating cell with a gas dispersion tube.
**FIG. 8** presents an electroplating system with constant voltage control or constant current control.
**FIG. 9** presents an electroplating system with pulsed current control.
**FIG. 10** presents an electroplating system with pulsed voltage control.
**FIG. 11** presents an electroplating system with scanned voltage control.
**FIG. 12** is a side view of a flex circuit that is bonded with adhesive to a hybrid substrate.
**FIG. 13** illustrates a flexible circuit being bonded using conductive metal pads to a hybrid substrate.
**FIG. 14** presents a ceramic substrate and flexible circuit with bond pads plated with platinum grey.
**FIG. 15** presents a ceramic substrate and flexible circuit with bond pads plated with platinum grey and bonded together with conductive epoxy.
**FIG. 16** presents a perspective with cutaway view of the implanted portion of the retinal prosthesis electrode array assembly showing the electronics package 2014.
**FIG. 17** is a side view of the implanted portion of the preferred retinal prosthesis showing the fan tail in more detail.
**FIG. 18** presents a view of the package showing its attachment to the flexible circuit electrode array.
**FIG. 19** presents a cut away view showing the inside of the electronics package.
**FIG. 20** depicts a cutaway view of an electronics package 14 showing the gold pads 78 and silicone overmold 90 with the ceramic substrate 60.
**FIG. 21** illustrates a cutaway view of the electronics package 14 of **FIG. 2** showing metalized vias 65 in the ceramic substrate 60.
**FIG. 22** depicts the ion beam assisted deposition apparatus with the ceramic substrate 104.
**FIG. 23** presents a cross-sectional view of ceramic substrate 202 and nanochannel vias 204 filled with wire 206 patterned with metal trace 222 and insulation layer 214.
**FIG. 24** is a cross-section of the braze joint.
**FIGS. 25A and B** present a package with an extended wall for impact resistance.
**FIGS. 26A and B** present a package with an extended wall and flange for impact resistance.
**FIGS. 27** **A and B** present a package with an extended wall and base for impact resistance.
**FIG. 28** presents the impact resistant package fully imbedded in a skull.
**FIG. 29** presents the impact resistant package partially imbedded in a skull.
**FIG. 30** presents the impact resistant package on the surface of the skull.

### DETAILED DESCRIPTION OF THE INVENTION

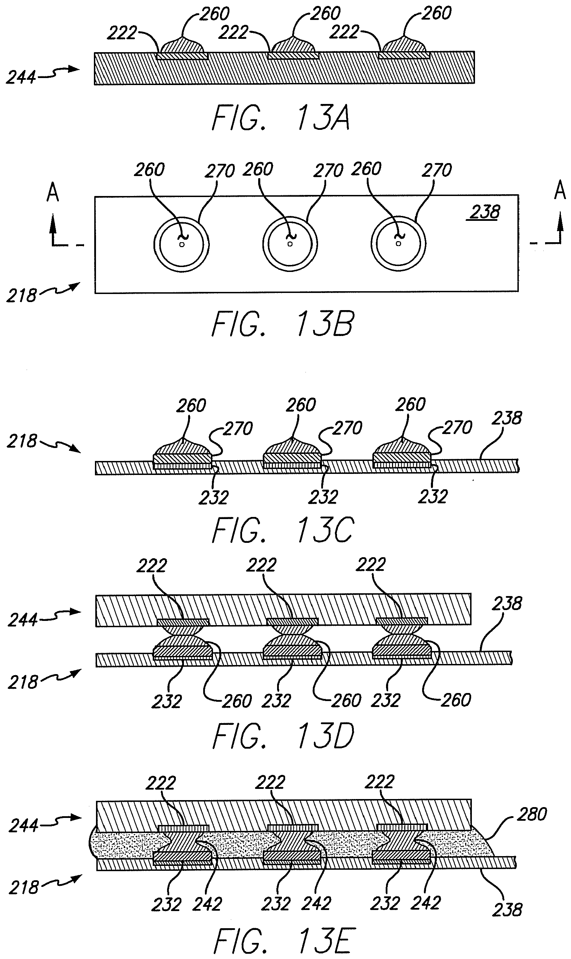

Referring to **FIG. 1**, an illustrative example of a platinum gray surface coating for an electrode is shown having a fractal surface with a surface area increase of greater than 5 times the surface area for a shiny platinum surface of the same geometry, shown in **FIG. 2**, and an increase in strength over a platinum black surface, shown in **FIG. 3. FIGS. 1**, **2**, and **3** are photomicrographs produced on a Scanning Electron Microscope (SEM) at 2000x magnification taken by a JEOL JSM5910 microscope (Tokyo, Japan). Under this magnification it is observed that platinum gray is a fractal configuration having a cauliflower shape with particle sizes ranging from 0.5 to 15 micrometers. Each branch of such structure is further covered by smaller and smaller particles of similar shape. The smallest particles on the surface layer may be in the nanometer range. This rough and porous fractal structure increases the electrochemically active surface area of the platinum surface when compared to an electrode with a smooth platinum surface having the same overall geometric shape and size.

The surface is pure platinum because no impurities or other additives such as lead need be introduced during the plating process to produce platinum gray. This is especially advantageous in the field of implantable electrodes because lead is neurotoxin and cannot be used in the process of preparing implantable electrodes. Alternatively, other materials such as iridium, rhodium, gold, tantalum, titanium or niobium could be introduced during the plating process, if desired, but these materials are not necessary to the formation of platinum gray.

Platinum gray can also be distinguished from platinum black and shiny platinum by measuring the color of the material on a spectrodensitometer using the Commission on Illumination l*a*b* color scale. l* defines lightness, a* denotes the red/green value and b*, the yellow/blue value. The lightness value (called l* Value) can range from 0 to 100, where white is 100 and black is 0, similar to grayscale. The a* value can range from +60 for red and -60 for green, and the b* value can range from +60 for yellow and -60 for blue. All samples measured have very small a* and b* values (they are colorless or in the so called neutral gray zone), which suggests that the lightness value can be used as grayscale for platinum coatings.

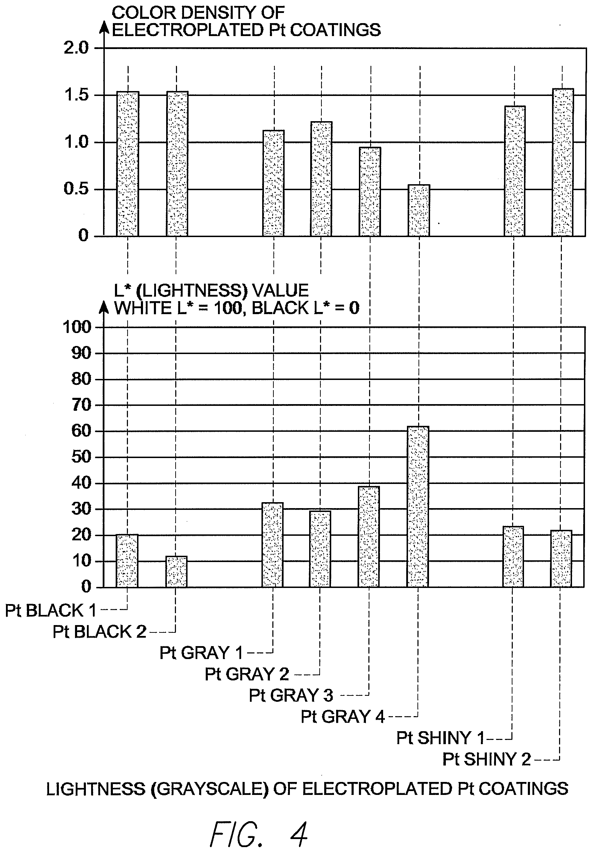

Referring to **FIG. 4**, the l*, a*, and b* values for representative samples of platinum gray, platinum black and shiny platinum are shown as measured on a color reflection spectrodensitometer, X-Rite 520. The l* value ranges from 25 to 90 for platinum gray, while platinum black and shiny platinum both have l* values less than 25.

Referring to **FIG. 4**, color densities have also been measured for representative samples of platinum gray, platinum black and shiny platinum. Platinum gray's color density values range from 0.4D to 1.3D; while platinum black and shiny platinum both have color density values greater than 1.3D.

Platinum gray can also be distinguished from platinum black based on the adhesive and strength properties of the thin film coating of the materials. Adhesion properties of thin film coatings of platinum gray and platinum black on 500 micrometers in diameter electrodes have been measured on a Micro-Scratch Tester (CSEM Instruments, Switzerland). A controlled micro-scratch is generated by drawing a spherical diamond tip of radius 10 micrometers across the coating surface under a progressive load from 1 millinewton to 100 millinewtons with a 400 micrometer scratch length. At a critical load the coating will start to fail. Using this test it was found that platinum gray can sustain a critical load of over 60 millinewtons while platinum black sustains a critical load of less than 35 millinewtons.

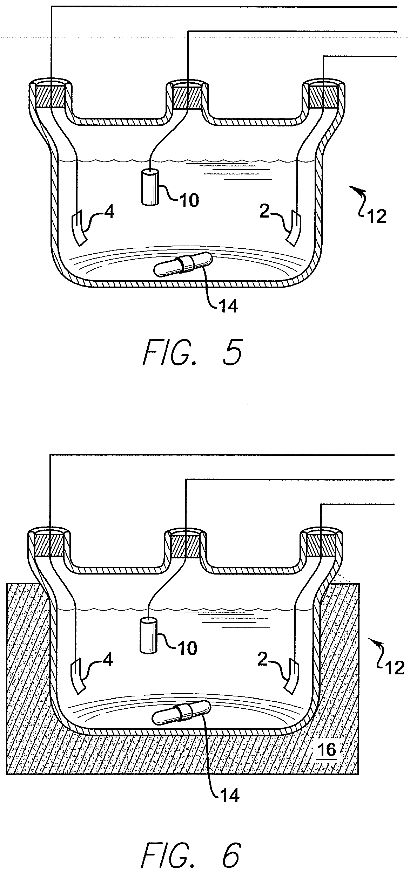

Referring to **FIGS. 5**, **6**, **7** and **8**, a method to produce platinum gray according to the present invention is described comprising connecting a platinum electrode 2, the anode, and a conductive substrate to be plated 4, the cathode, to a power source 6 with a means of controlling and monitoring 8 either the current or voltage of the power source 6. The anode 2, cathode 4, a reference electrode 10 for use as a reference in controlling the power source 6 and an electroplating solution are placed in a electroplating cell 12 having a means 14 for mixing or agitating the electroplating solution. Power is supplied to the electrodes with constant voltage, constant current, pulsed voltage, scanned voltage or pulsed current to drive the electroplating process. The power source 6 is modified such that the rate of deposition will cause the platinum to deposit as platinum gray, the rate being greater than the deposition rate necessary to form shiny platinum and less than the deposition rate necessary to form platinum black.

Referring to **FIGS. 5**, **6** and **7**, the electroplating cell 12, is preferably a 50 to 150 ml four neck glass flask or beaker, the common electrode 2, or anode, is preferably a large surface area platinum wire or platinum sheet, the reference electrode 10 is preferably a Ag/AgCI electrode (silver, silver chloride electrode), the conductive substrate to be plated 4, or cathode, can be any suitable material depending on the application and can be readily chosen by one skilled in the art. Preferable examples of the conductive substrate to be plated 4 include but are not limited to platinum, iridium, rhodium, gold, tantalum, titanium or niobium.

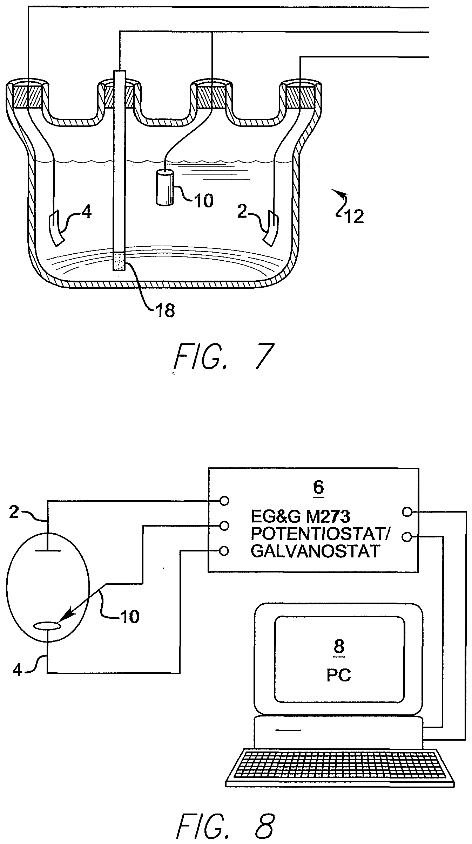

The stirring mechanism is preferably a magnetic stirrer 14 as shown in **FIG. 5**, an ultrasonic tank 16 (such as the VWR Aquasonic 50D) as shown in **FIG. 6**, or gas dispersion 18 with Argon or Nitrogen gas as shown in **FIG. 7**. The plating solution is preferably 3 to 30 mM (milimole) ammonium hexachloroplatinate in disodium hydrogen phosphate, but may be derived from any chloroplatinic acid or bromoplatinic acid or other electroplating solution. The preferable plating temperature is approximately 24° to 26°C.

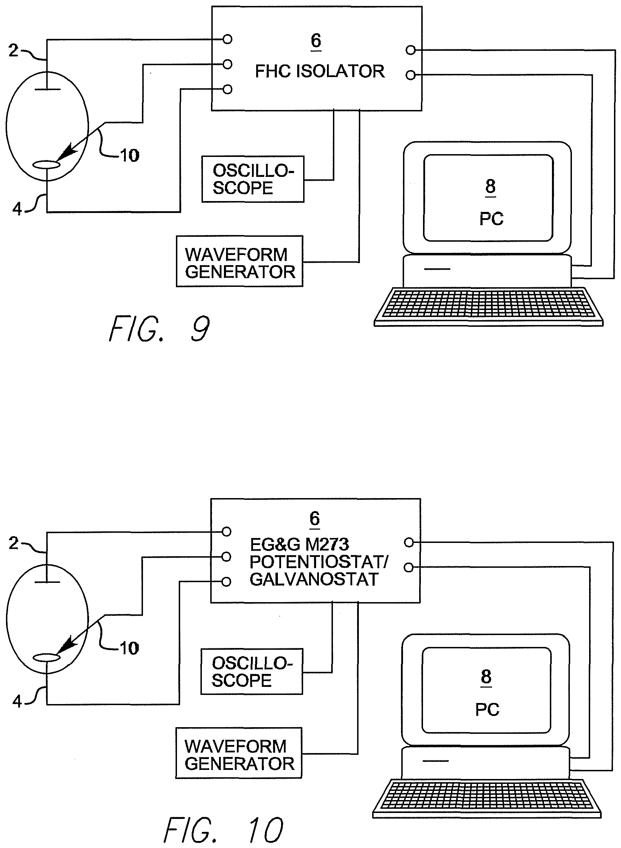

Electroplating systems with pulsed current and pulsed voltage control are shown in **FIGS. 9** and **10** respectively. While constant voltage, constant current, pulsed voltage or pulsed current can be used to control the electroplating process, constant voltage control of the plating process has been found to be most preferable. The most preferable voltage range to produce platinum gray is - 0.45 Volts to -0.85 Volts. Applying voltage in this range with the above solution yields a plating rate in the range of about 1.0 to 0.05 micrometers per minute, the preferred range for the plating rate of platinum gray. Constant voltage control also allows an array of electrodes in parallel to be plated simultaneously achieving a fairly uniform surface layer thickness for each electrode.

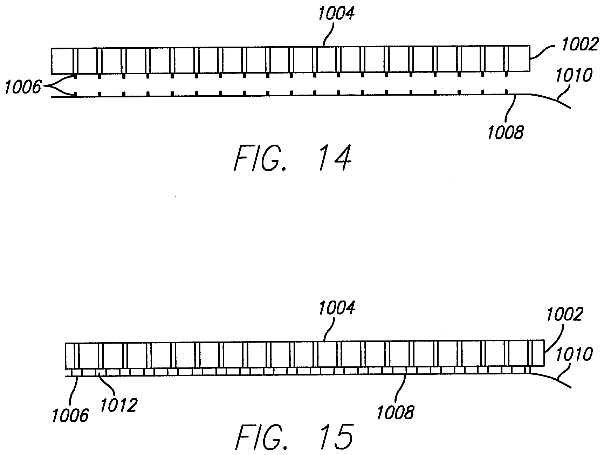

The optimal potential ranges for platinum gray plating are solution and condition dependent. Linear voltage sweep can be used to determine the optimal potential ranges for a specific plating system. A representative linear voltage sweep is presented, **FIG. 14**. During linear voltage sweep, the voltage of an electrode is scanned cathodically until hydrogen gas evolution occurs which reveals plating rate control steps of electron transfer 20 and diffusion 22. For a given plating system, it is preferable to adjust the electrode potential such that the platinum reduction reaction has a limiting current under diffusion control or mixed control 24 between diffusion and electron transfer but that does not result in hydrogen evolution 26.

It has been found that because of the physical strength of platinum gray, surface layers of thickness greater than 30 micrometers can be plated. It is very difficult to plate shiny platinum in layers greater than approximately several micrometers because the internal stress of the dense platinum layer which will cause the plated layer to peel off and the underlying layers cannot support the above material. The additional thickness of the plate's surface layer allows the electrode to have a much longer usable life.

The following example is illustrative of electroplating platinum on a conductive substrate to form a surface coating of platinum gray.

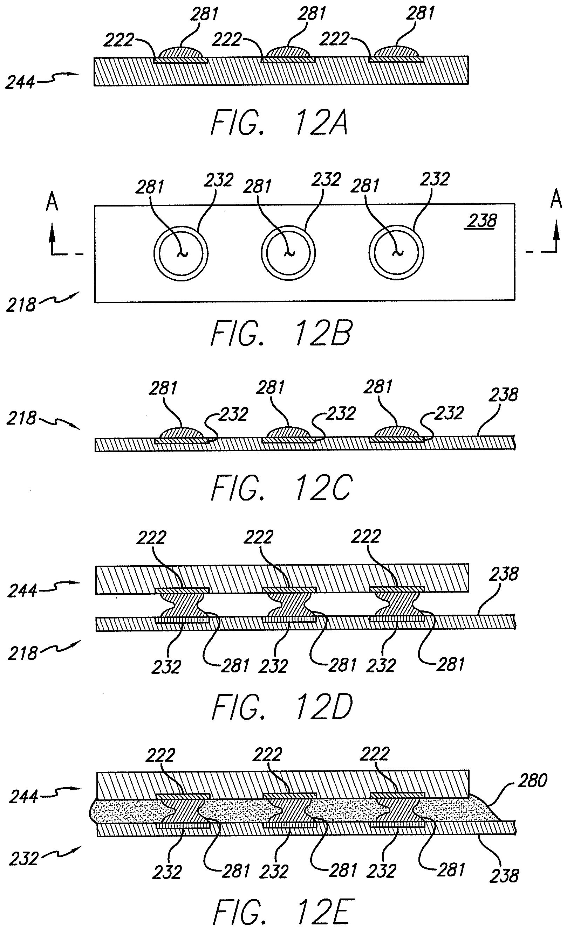

Electrodes with a surface layer of platinum gray are prepared in the following manner using constant voltage plating. An electrode platinum silicone array having 16 electrodes where the diameter of the platinum discs on the array range from 510 to 530 micrometers, as shown in **FIG. 12**, is first cleaned electrochemically in sulfuric acid and the starting electrode impedance is measured in phosphate buffered saline solution. Referring to **FIG. 5**, the electrodes are arranged in the electroplating cell such that the plating electrode 2 is in parallel with the common electrode 4. The reference electrode 10 is positioned next to the electrode 4. The plating solution is added to the electroplating cell 12 and the stirring mechanism 14 is activated.

A constant voltage is applied on the plating electrode 2 as compared to the reference electrode 10 using an EG&G PAR M273 potentiostat 6. The response current of the plating electrode 2 is recorded by a recording means 8. (The response current is measured by the M273 potentiostat 6.) After a specified time, preferably 1 to 90 minutes, and most preferably 30 minutes, the voltage is terminated and the electrode 4 is thoroughly rinsed in deionized water.

The electrochemical impedance of the electrode array with the surface coating of platinum gray is measured in a saline solution. The charge/charge density and average plating current/current density are calculated by integrating the area under the plating current vs. time curve. Scanning Electron Microscope (SEM)/ Energy Dispersed Analysis by X-ray (EDAX™) analysis can be performed on selected electrodes. SEM photomicrographs of the plated surface show its fractal surface. Energy dispersed Analysis demonstrates that the sample is pure platinum rather than platinum oxide or some other materials.

From this example it is observed that the voltage range is most determinative of the formation of the fractal surface of platinum gray. For this system it observed that the optimal voltage drop across the electrodes to produce platinum gray is approximately -0.55 to -0.65 volts vs. Ag/AgCl reference electrode 10. The optimal platinum concentration for the plating solution is observed to be approximately 8 to 18 mM ammonium hexachloroplatinate in 0.4 M (Mole) disodium hydrogen phosphate.

### Platinum Conductor in Polymer Adhesive

A preferred embodiment of the invention, illustrated in **FIG. 12**, shows the method of bonding the hybrid substrate 244 to the flexible circuit 218 using electrically conductive adhesive 281, such as a polymer, which may include polystyrene, epoxy, or polyimide, which contains electrically conductive particulate of select biocompatible metal, such as platinum, iridium, titanium, rhodium, gold, tantalum, titanium or niobium, or any alloy thereof, in dust, flake, or powder form.

In **FIG. 12**, step a, the hybrid substrate 244, which may alternatively be an integrated circuit or electronic array, and the input/output contacts 222 are prepared for bonding by placing conductive adhesive 281 on the input/output contacts 222. The rigid integrated circuit 244 is preferably comprised of a ceramic, such as zirconia, or silicon. In step b, the flexible circuit 218 is preferably prepared for bonding to the hybrid substrate 244 by placing conductive adhesive 281 on bond pads 232. Alternatively, the adhesive may be coated with an electrically conductive biocompatible metal. The flexible circuit 218 contains the flexible electrically insulating substrate 238, which is preferably comprised of polyimide, or another biocompatible polymer. The bond pads 232 are preferably comprised of an electrically conductive material that is biocompatible when implanted in living tissue, and are preferably platinum or a platinum alloy, such as platinum-iridium.

**FIG. 12**, step c illustrates the cross-sectional view A-A of step b. The conductive adhesive 281 is shown in contact with and resting on the bond pads 232. Step d shows the hybrid substrate 244 in position after being bonded to the flexible circuit 218. The conductive adhesive 281 provides an electrical path between the input/output contacts 222 and the bond pads 232. Step e illustrates the completed bonded assembly wherein the flexible circuit 218 is bonded to the hybrid substrate 144, thereby providing a path for electrical signals to pass to the living tissue from the electronics control unit (not illustrated). The assembly has been electrically isolated and sealed with adhesive underfill 280, which is preferably epoxy.

### Studbump Bonding

**FIG. 13** illustrates the steps of an alternative embodiment to bond the hybrid substrate 244 to flexible circuit 218 by studbumping the hybrid substrate 244 and flexible electrically insulating substrate 238 prior to bonding the two components together by a combination of heat and/or pressure, such as ultrasonic energy. In step a, the hybrid substrate 244 is prepared for bonding by forming a studbump 260 on the input/output contacts 222. The studbump is formed by known methods and is preferably comprised of an electrically conductive material that is biocompatible when implanted in living tissue if exposed to a saline environment. It is preferably comprised of metal, preferably biocompatible metal, or gold or of gold alloys. If gold is selected, then it must be protected with a water resistant adhesive or underfill 280.

Alternatively, the studbump 260 may be comprised of an insulating material, such as an adhesive or a polymer, which is coated with an electrically conductive coating of a material that is biocompatible and stable when implanted in living tissue, while an electric current is passed through the studbump 260. One such material coating may preferably be platinum or alloys of platinum, such as platinum-iridium, where the coating may be deposited by vapor deposition, such as by ion-beam assisted deposition, or electrochemical means.

**FIG. 13**, step b presents the flexible circuit 218, which comprises the flexible electrically insulating substrate 238 and bond pads 232. The flexible circuit 218 is prepared for bonding by the plating bond pads 232 with an electrically conductive material that is biocompatible when implanted in living tissue, such as with a coating of platinum or a platinum alloy. Studbumps 260 are then formed on the plated pad 270 by known methods. Step c illustrates cross-section A-A of step b, wherein the flexible circuit 218 is ready to be mated with the hybrid substrate 244.

**FIG. 13**, step d illustrates the assembly of hybrid substrate 244 flipped and ready to be bonded to flexible circuit 218. Prior to bonding, the studbumps 260 on either side may be flattened by known techniques such as coining. Pressure is applied to urge the mated studbumps 260 together as heat is applied to cause the studbumps to bond by a diffusion or a melting process. The bond may preferably be achieved by thermosonic or thermocompression bonding, yielding a strong, electrically conductive bonded connection 242, as illustrated in step e. An example of a thermosonic bonding method is ultrasound. The bonded assembly is completed by placing an adhesive underfill 280 between the flexible circuit 218 and the hybrid substrate 244, also increasing the strength of the bonded assembly and electrically isolating each bonded connection. The adhesive underfill 280 is preferably epoxy.

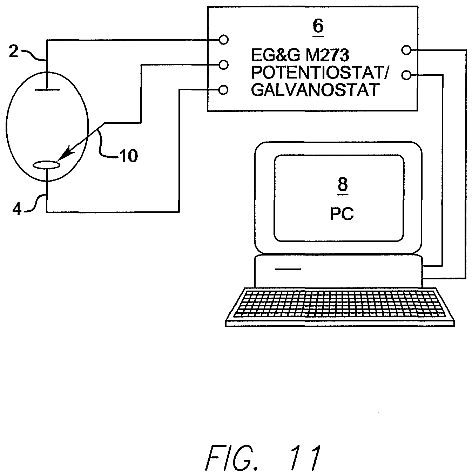

**FIG. 14** shows the interconnection of the present invention in further detail. The interconnection process as described with respect to **FIGS. 12** and **13** can be improved with the electroplated platinum gray as described with respect to **FIGS. 1** to **11**. The ceramic substrate 1002 includes platinum vias 1004. The vias 1004 align with bond pads 1008 on the flexible circuit 1010. Alternatively, metal traces may be applied to the lower surface of the ceramic substrate 1002 allowing bond pads in different locations than the terminus of the vias 1004. Platinum gray 1006 may be platted on the vias 1004 or on the bond pads if metal traces are used. Platinum gray 1006 may also be platted on the bond pads 1008 of the flexible circuit and/or on the bond pads of the ceramic substrate 1002.

**FIG. 15** shows the flexible circuit 1010 connected to the ceramic substrate 1002 with conductive epoxy 1012 covering the platinum gray 1006. Underfill (not shown) may be further applied, **FIGS. 12** and **13**. While platinum gray is the preferred method of achieving a roughen surface to improved adhesion with the conductive epoxy, many other methods are suitable for obtaining a roughen surface. Other methods include sandblasting, reactive ion etching (RIE), thin-film coating of an adhesion layer by chemical vapor deposition, physical vapor deposition, atomic layer deposition, or other deposition techniques. Adhesion may be further enhanced by roughening the surface of the ceramic substate 1002 and/or flexible circuit 1010. Roughening these surfaces will also increase adhesion of the underfill. Sandblasting or RIE are effective methods of roughening ceramic or polymer.

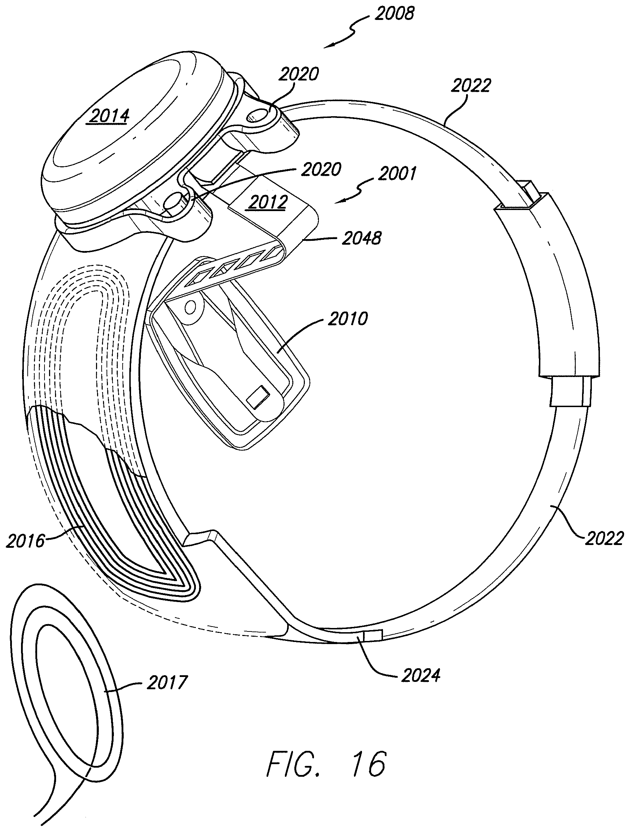

**FIG. 16** shows a perspective view of the implanted portion of the preferred visual prosthesis. A flexible circuit 2001 includes a flexible circuit electrode array 2010 which is mounted by a retinal tack (not shown) or similar means to the epiretinal surface. The flexible circuit electrode array 2010 is electrically coupled by a flexible circuit cable 2012, which pierces the sclera and is electrically coupled to an electronics package 2014, external to the sclera.

The electronics package 2014 is electrically coupled to a secondary inductive coil 2016. Preferably the secondary inductive coil 2016 is made from wound wire. Alternatively, the secondary inductive coil 2016 may be made from a flexible circuit polymer sandwich with wire traces deposited between layers of flexible circuit polymer. The secondary inductive coil receives power and data from a primary inductive coil 2017, which is external to the body. The electronics package 2014 and secondary inductive coil 2016 are held together by the molded body 2018. The molded body 18 holds the electronics package 2014 and secondary inductive coil 16 end to end. The secondary inductive coil 16 is placed around the electronics package 2014 in the molded body 2018. The molded body 2018 holds the secondary inductive coil 2016 and electronics package 2014 in the end to end orientation and minimizes the thickness or height above the sclera of the entire device. The molded body 2018 may also include suture tabs 2020. The molded body 2018 narrows to form a strap 2022 which surrounds the sclera and holds the molded body 2018, secondary inductive coil 2016, and electronics package 2014 in place. The molded body 2018, suture tabs 2020 and strap 2022 are preferably an integrated unit made of silicone elastomer. Silicone elastomer can be formed in a pre-curved shape to match the curvature of a typical sclera. However, silicone remains flexible enough to accommodate implantation and to adapt to variations in the curvature of an individual sclera. The secondary inductive coil 2016 and molded body 2018 are preferably oval shaped. A strap 2022 can better support an oval shaped coil. It should be noted that the entire implant is attached to and supported by the sclera. An eye moves constantly. The eye moves to scan a scene and also has a jitter motion to improve acuity. Even though such motion is useless in the blind, it often continues long after a person has lost their sight. By placing the device under the rectus muscles with the electronics package in an area of fatty tissue between the rectus muscles, eye motion does not cause any flexing which might fatigue, and eventually damage, the device.

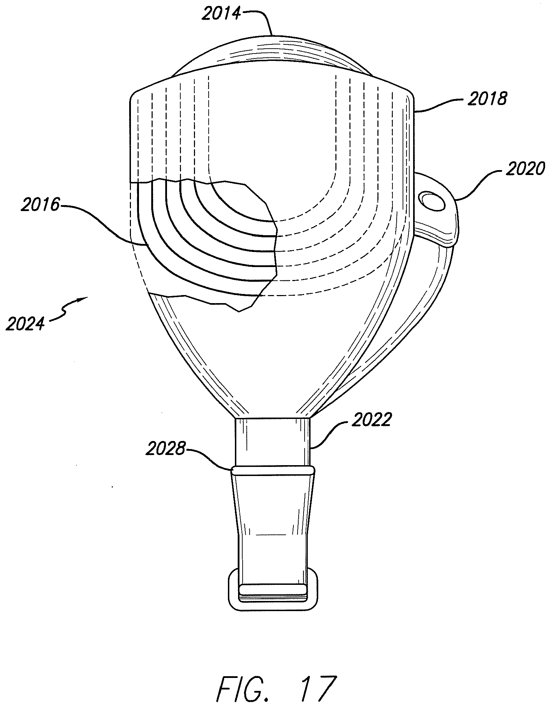

**FIG. 17** shows a side view of the implanted portion of the visual prosthesis, in particular, emphasizing the fan tail 2024. When implanting the visual prosthesis, it is necessary to pass the strap 2022 under the eye muscles to surround the sclera. The secondary inductive coil 2016 and molded body 2018 must also follow the strap 2022 under the lateral rectus muscle on the side of the sclera. The implanted portion of the visual prosthesis is very delicate. It is easy to tear the molded body 2018 or break wires in the secondary inductive coil 2016. In order to allow the molded body 18 to slide smoothly under the lateral rectus muscle, the molded body 2018 is shaped in the form of a fan tail 2024 on the end opposite the electronics package 2014. The strap 2022 further includes a hook 2028 the aids the surgeon in passing the strap under the rectus muscles.

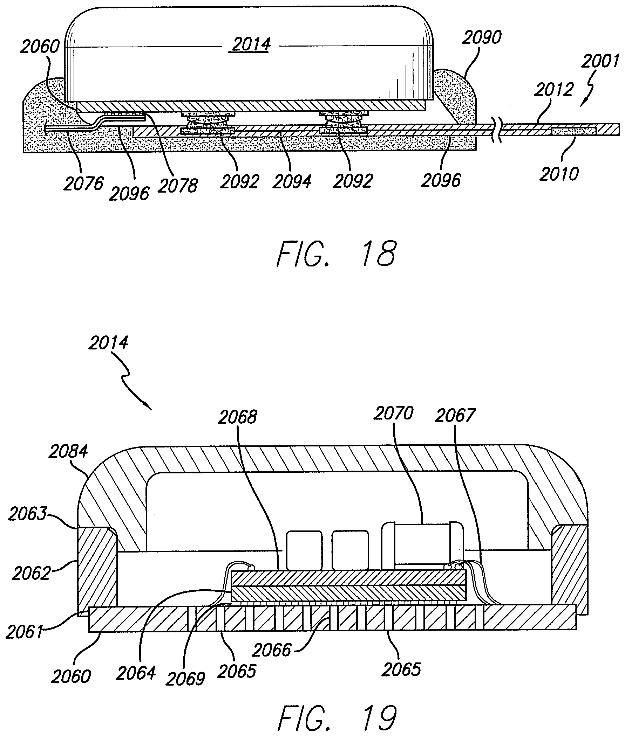

Referring to **FIG. 18**, the flexible circuit 2001, includes platinum conductors 2094 insulated from each other and the external environment by a biocompatible dielectric polymer 2096, preferably polyimide. One end of the array contains exposed electrode sites that are placed in close proximity to the retinal surface. The other end contains bond pads 2092 that permit electrical connection to the electronics package 2014. The electronic package 2014 is attached to the flexible circuit 2001 using a flip-chip bumping process, and epoxy underfilled. In the flip-chip bumping process, bumps containing conductive adhesive placed on bond pads 2092 and bumps containing conductive adhesive placed on the electronic package 2014 are aligned and melted to build a conductive connection between the bond pads 2092 and the electronic package 2014. Leads 2076 for the secondary inductive coil 2016 are attached to gold pads 2078 on the ceramic substrate 2060 using thermal compression bonding, and are then covered in epoxy. The electrode array cable 2012 is laser welded to the assembly junction and underfilled with epoxy. The junction of the secondary inductive coil 2016, flexible circuit 2001, and electronic package 2014 are encapsulated with a silicone overmold 2090 that connects them together mechanically. When assembled, the hermetic electronics package 2014 sits about 3 mm away from the end of the secondary inductive coil.

Since the implant device is implanted just under the conjunctiva it is possible to irritate or even erode through the conjunctiva. Eroding through the conjunctiva leaves the body open to infection. We can do several things to lessen the likelihood of conjunctiva irritation or erosion. First, it is important to keep the over all thickness of the implant to a minimum. Even though it is advantageous to mount both the electronics package 2014 and the secondary inductive coil 2016 on the lateral side of the sclera, the electronics package 2014 is mounted higher than, but not covering, the secondary inductive coil 2016. In other words the thickness of the secondary inductive coil 2016 and electronics package should not be cumulative.

It is also advantageous to place protective material between the implant device and the conjunctiva. This is particularly important at the sclerotomy, where the thin film electrode array cable 2012 penetrates the sclera. The thin film electrode array cable 2012 must penetrate the sclera through the pars plana, not the retina. The sclerotomy is, therefore, the point where the device comes closest to the conjunctiva. The protective material can be provided as a flap attached to the implant device or a separate piece placed by the surgeon at the time of implantation. Further material over the sclerotomy will promote healing and sealing of the sclerotomy. Suitable materials include DACRON®, TEFLON®, GORETEX® (ePTFE), TUTOPLAST® (sterilized sclera), MERSILENE® (polyester) or silicone.

Referring to **FIG. 19**, the package 2014 contains a ceramic substrate 2060, with metalized vias 2065 and thin-film metallization 2066. The package 2014 contains a metal case wall 2062 which is connected to the ceramic substrate 2060 by braze joint 2061. On the ceramic substrate 2060 an underfill 2069 is applied. On the underfill 69 an integrated circuit chip 2064 is positioned. On the integrated circuit chip 2064 a ceramic hybrid substrate 2068 is positioned. On the ceramic hybrid substrate 2068 passives 2070 are placed. Wirebonds 2067 are leading from the ceramic substrate 2060 to the ceramic hybrid substrate 2068. A metal lid 2084 is connected to the metal case wall 2062 by laser welded joint 2063 whereby the package 2014 is sealed.

Accordingly, what has been shown is an improved visual prosthesis and an improved method for limiting power consumption in a visual prosthesis. While the invention has been described by means of specific embodiments and applications thereof, it is understood that numerous modifications and variations could be made thereto by those skilled in the art without departing from the scope of the invention. It is therefore to be understood that within the scope of the claims, the invention may be practiced otherwise than as specifically described herein.

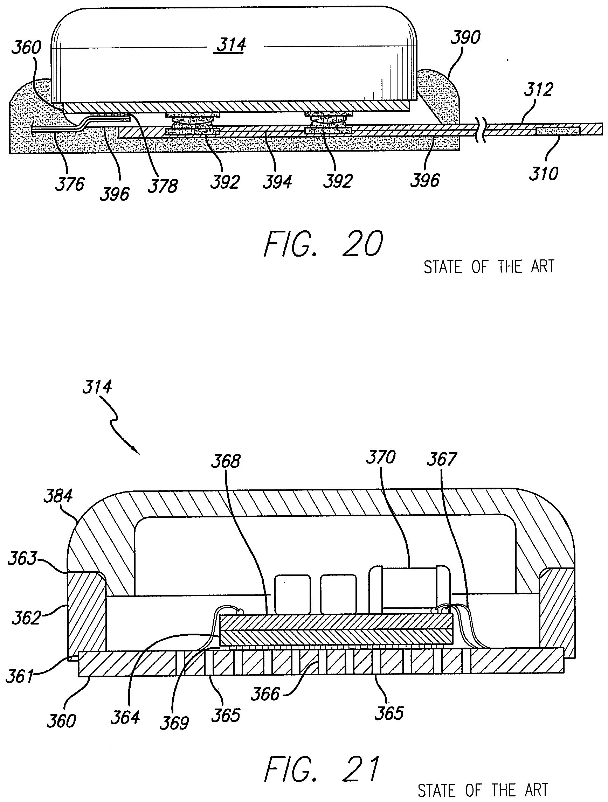

Referring to **FIG. 20**, the flexible circuit 2001, of **FIG. 16**, includes platinum conductors 394 insulated from each other and the external environment by a biocompatible dielectric polymer 396, preferably polyimide. One end of the array contains exposed electrode sites that are placed in close proximity to the retinal surface 310. The other end contains bond pads 392 that permit electrical connection to the electronics package 314. The electronic package 314 is attached to the flexible circuit 2001 using a flip-chip bumping process, and epoxy underfilled. In the flip-chip bumping process, bumps containing conductive adhesive placed on bond pads 392 and bumps containing conductive adhesive placed on the electronic package 314 are aligned and melted to build a conductive connection between the bond pads 392 and the electronic package 314. Leads 376 for the secondary inductive coil 316 are attached to gold pads 378 on the ceramic substrate 360 using thermal compression bonding, and are then covered in epoxy. The electrode array cable 312 is laser welded to the assembly junction and underfilled with epoxy. The junction of the secondary inductive coil 16, array, and electronic package 314 are encapsulated with a silicone overmold 390 that connects them together mechanically. When assembled, the hermetic electronics package 314 sits about 3 mm away from the end of the secondary inductive coil.

Since the implant device is implanted just under the conjunctiva it is possible to irritate or even erode through the conjunctiva. Eroding through the conjunctiva leaves the body open to infection. We can do several things to lessen the likelihood of conjunctiva irritation or erosion. First, it is important to keep the over all thickness of the implant to a minimum. Even though it is advantageous to mount both the electronics package 314 and the secondary inductive coil 316 on the lateral side of the sclera, the electronics package 314 is mounted higher than, but not covering, the secondary inductive coil 316. In other words the thickness of the secondary inductive coil 316 and electronics package should not be cumulative.

It is also advantageous to place protective material between the implant device and the conjunctiva. This is particularly important at the sclerotomy, where the thin film electrode array cable 312 penetrates the sclera. The thin film electrode array cable 312 must penetrate the sclera through the pars plana, not the retina. The sclerotomy is, therefore, the point where the device comes closest to the conjunctiva. The protective material can be provided as a flap attached to the implant device or a separate piece placed by the surgeon at the time of implantation. Further material over the sclerotomy will promote healing and sealing of the sclerotomy. Suitable materials include DACRON®, TEFLON®, GORETEX® (ePTFE), TUTOPLAST® (sterilized sclera), MERSILENE® (polyester) or silicone.

Referring to **FIG. 21**, the package 314 contains a ceramic substrate 360, with metalized vias 365 and thin-film metallization 366. The package 314 contains a metal case wall 362 which is connected to the ceramic substrate 360 by braze joint 361. On the ceramic substrate 360 an underfill 369 is applied. On the underfill 369 an integrated circuit chip 364 is positioned. On the integrated circuit chip 364 a ceramic hybrid substrate 68 is positioned. On the ceramic hybrid substrate 368 passive electronics 370 are placed. Wirebonds 367 lead from the ceramic substrate 360 to the ceramic hybrid substrate 368. A metal lid 384 is connected to the metal case wall 362 by laser welded joint 363 whereby the package 314 is sealed.

While there are several known techniques for depositing a thick film metal trace 222 or an insulation layer 214 by sputtering or physical vapor deposition, for example, ion beam assisted deposition (IBAD) is a preferred method.

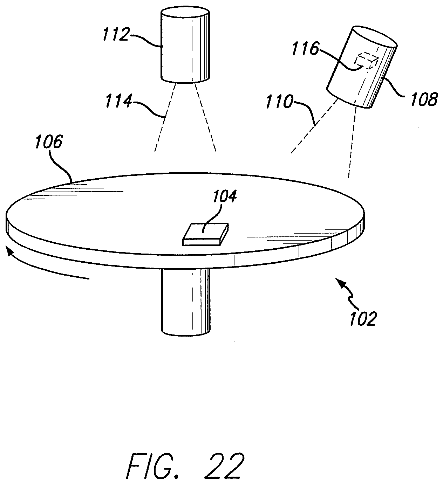

The ceramic substrate 202 is formed into a desired final shape and is then coated with the desired thick film 222, 214, such as alumina, by the ion beam assisted deposition process of **FIG. 22**. The IBAD process creates an adherent layer of alumina. The resulting alumina coating is dense and strongly adherent to the ceramic substrate 202. Alternate deposition methods are known, including magnetron sputter deposition and ion implantation coating deposition.

In a preferred embodiment, the coating thickness is at least about 1.6 micrometers. If the coating thickness is greater than about 10 micrometers, then the coating is more likely to crack or spall off of the substrate. The average grain size of the alumina is preferably less than about 0.5 micrometer average, as measured by the line intersection method. This increases the toughness of the coating.

The IBAD process apparatus 2, **FIG. 22**, involves placing a substrate 104, which is also often referred to as the "target", to be coated on a substrate holder 106. The substrate is heated to about 300°C. The substrate holder 106 preferably rotates slowly at about one revolution per minute, to assist in obtaining a uniformly thick and dense coating on substrate 104. An ion gun 108, substrate holder 106, and e-beam evaporator 112 are located near the substrate in an environmentally controlled chamber, which is preferably a vacuum chamber that allows an inert gas, preferably argon, to be backfilled into the chamber with a small amount of oxygen. In alternate embodiments, other inert gases, such as nitrogen, or mixtures of inert gases may be utilized in combination with oxygen. In a preferred embodiment, there are two sources of argon; one to the ion gun and one to the IBAD chamber.

The ion gun 108 includes a source of the desired coating, preferably an alumina source 16, in a preferred embodiment. An ion beam 110 is generated wherein the energetic ions of alumina are directed toward the substrate 104. Simultaneously and continuously with the release of the ions, the e-beam evaporator 112 bombards the substrate 104 and the alumina coating, as it is forming, with an electron beam 114 that is emitted by a heated tungsten filament. It is preferred that the alumina coating be comprised of alpha-alumina or amorphous alumina. Because alpha-alumina is stronger, harder, and has a higher specific gravity than other aluminas, including amorphous alumina, alpha-alumina is a preferred phase. Amorphous alumina may be converted to alpha-alumina by annealing at about 1000°C. The IBAD process yields both amorphous alumina and alpha alumina in proportions that are dictated by the deposition parameters. A blend of alpha-alumina and amorphous alumina results under certain deposition parameters. It is believed that rapid quenching of the vapor phase results in a predominance of amorphous alumina. Therefore, control of the deposition parameters allows the preferred alpha-alumina phase to be formed in the coating on substrate 104.

It is known to those skilled in the art that the resulting coating has a high bulk density, comprising very low open or closed porosity, preferably less than 1.0% total porosity. Therefore, the alumina coating offers excellent resistance to moisture penetration, thereby eliminating or dramatically reducing moisture penetration and diffusion to the substrate 104.

### Example

The base vacuum level is about 1x10⁻⁷ Torr and the working pressure of argon plus oxygen is about 3x10⁻⁴ Torr. In a chamber of approximately one gallon in volume, the flow rates to the ion gun 108 of the argon-oxygen mixture about 10 scc/m argon plus 5.5 scc/m oxygen. The flow rates to the IBAD chamber are about 5.5 scc/m oxygen and about 3.5 scc/m of argon.

The substrate temperature is about 300°C. The electron beam evaporation source is a solid, dense block of single crystal sapphire alumina with a purity of at least about 99.99 atomic percent.

The deposition rate is about 1.5 angstroms per second at an ion beam bombardment energy of about 1000 eV and an ion beam current of about 26 mA. In alternate embodiments, the film is bombarded with ions from an ion gun with energies typically in the range of 1.0 to 1.5 Kev. As a result, energy is transferred to the coating atoms, allowing them to migrate on the surface, and the coating can grow in a more uniform manner.

A 1.6 micormeters thick alumina coating was applied by IBAD on a sealed ceramic case comprised of alumina.

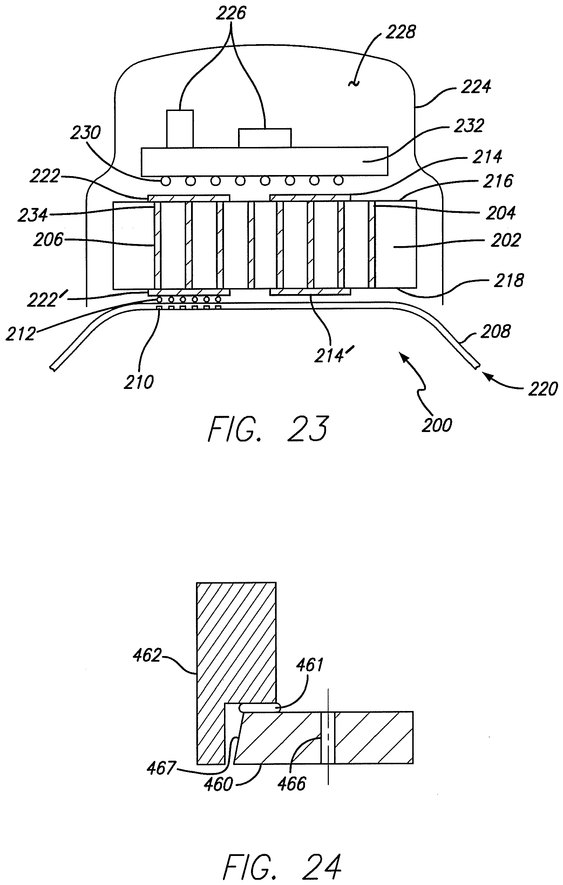

The improved living tissue implantable nanochannel device and microchip package 200 are presented in **FIG. 23**. Whalen teaches a device having closely arranged one micrometer diameter nanochannels 204 with conductive wires [or wire vias] 206 therethrough which terminate in microelectrodes 234 at the top surface 216 and bottom surface 218 of a ceramic substrate 202, thus forming an array of electrodes 234. The resulting electrodes 234 are difficult to isolate or to select individually. The close arrangement of vias and Whalen's pattern of metal prevents useful patterning.

A metal trace 222 if on the top surface 216 of the ceramic substrate 202 or metal trace 222' if on the bottom surface 218 of the substrate 202 is deposited by vapor deposition, such as the discussed IBAD, although sputtered deposition also is applicable. The metal trace 222 may be comprised of platinum or a platinum alloy, or noble metal alloy, for example.

The insulation layer 214 if on the top surface or 214' if on the bottom surface, is deposited in a manner and by a technique suitable for the metal trace 222 deposition, preferably IBAD.

This technique of selecting a group of nanochannels 204 for electrical conductors (vias) and the remainder of nanochannels being isolated by applying an insulation layer 214, which may be applied to either one or both the top surface 216 and or the bottom surface 218 of ceramic substrate 202. The insulation layer 214, 214' provides an over pattern on the vias to electrically isolate the vias from the metal layer, saline, or other components. The metal traces provide an electric connection of multiple vias together. Advantageously, this nanochannel metallization and insulation technique lowers impedance and redirects or maps to the flex circuit 220 or to internal electronics 226.

Looking at **FIG. 23**, a hermetic enclosure 228 is formed by hermetically bonding the metal can, preferably comprised of titanium or its alloys, to the ceramic substrate 202, which in a preferred embodiment is formed of alumina, although other ceramic oxides or dielectrics may be used. The hermetic enclosure protects the electronics 226, including resistors, capacitors, inductors, power supply, and diodes, from the surrounding environment, which for an implantable device is living tissue, a hostile warm saline environment.

The electronics are bonded to a circuit board 232. In this drawing gold bumps 230 are employed to bump bond to the vias by the metal trace 222 on the top surface 216 of the ceramic substrate 202. The ceramic substrate 202 contains a large number of nominal diameter one micrometer nanochannels 204 which are filled with a hermetically bonded wire 206, which is preferably an inert electrical conductor such as platinum or an alloy of platinum.

The bond pad may be remapped with a trace comprising a preferred layer of titanium, covered by platinum, and lastly covered by gold

The flex circuit 208, preferably comprised of polymer, such as polyimide or parylene, is bump bonded by bond pad 212 to the metal trace 222'. The flex circuit may be bonded by conductive epoxy on the bottom side 218. The flex circuit may be photolithographically patterned and etched or laser-write etched. Insulating layer 214' isolates the unneeded nanochannels and the wires contained therein from contacting the functioning electronics and thus interfering with the sensing or stimulation function of the microchip package 200.

Openings 210 are shown in the flex circuit to facilitate bonding while the electrode end 220 is shown leading to neuro or retinal contact electrodes, for example.

Referring to **FIG. 19**, the package 2014 contains a ceramic substrate 2060, with metallized vias 2065 and thin-film metallization 2066. The package 2014 contains a metal case wall 2062 which is connected to the ceramic substrate 2060 by braze joint 2061. On the ceramic substrate 2060 an underfill 2069 is applied. On the underfill 2069 an integrated circuit chip 2064 is positioned. On the integrated circuit chip 2064 a ceramic hybrid substrate 68 is positioned. On the ceramic hybrid substrate 2068 passives 2070 are placed. Wirebonds 2067 lead from the ceramic substrate 2060 to the ceramic hybrid substrate 2068. A metal lid 2084 is connected to the metal case wall 2062 by laser welded joint 2063 whereby the package 2014 is sealed.

Referring to **FIG. 24**, the braze joint of **FIG. 19** is shown in detail. The substrate 460 is laser cut to provide accurate alignment between the vias 466 and the edge of the substrate 467. However, a laser cut tends to create a slight bevel on the edge 467 of substrate 460. The bevel is slight and presented in **FIG. 24**. It is important to assemble the package with the smaller side of substrate 460 facing the metal case wall 462. It is further important that the braze 461 is on the top of the substrate 460 rather than against the edge 467. This allows gravity to help flow the braze material evenly and allows for inspection of the braze joint. The braze 461 is preferably a ring of titanium and nickel. Heating the assembly melts the titanium nickel ring without damaging the assembly.

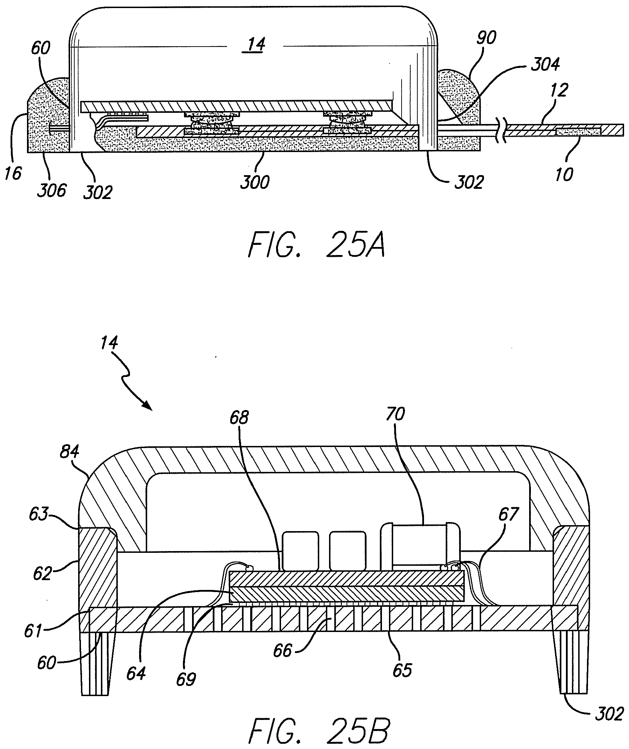

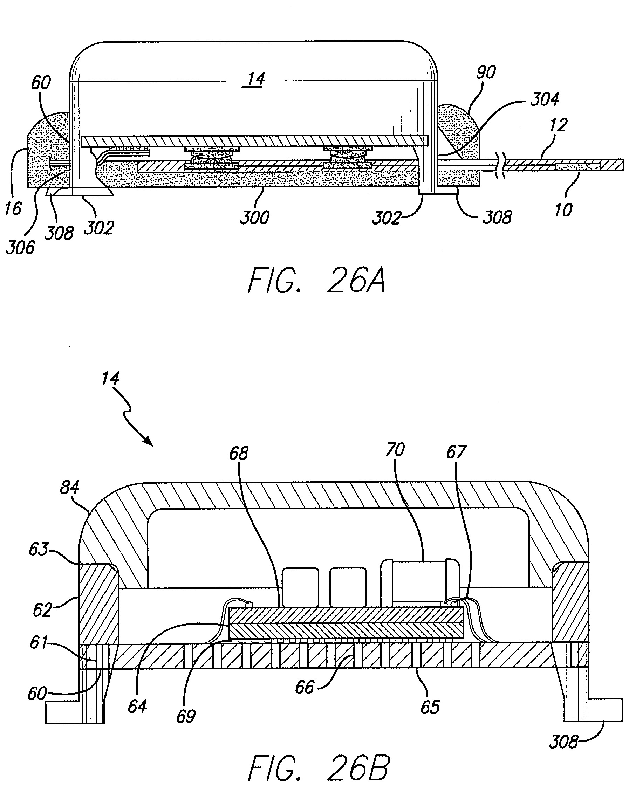

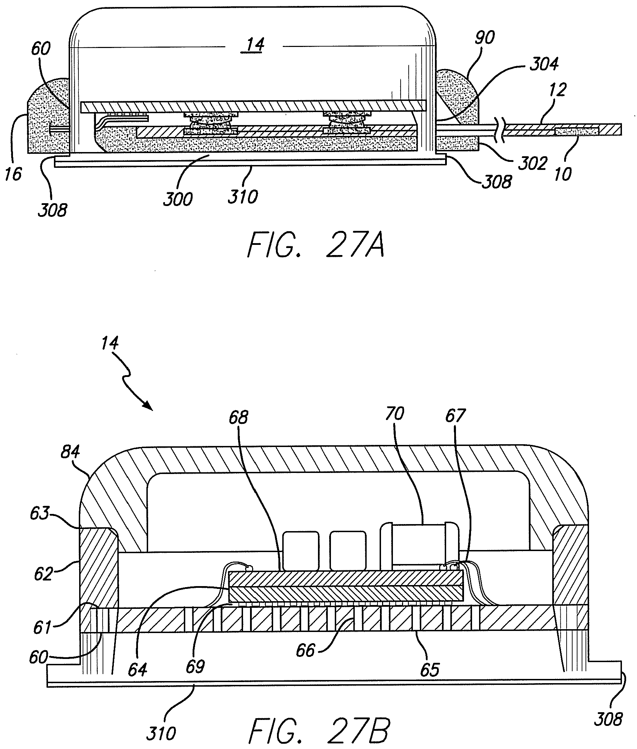

An alternate embodiment is presented in **FIGS. 25 - 30**. The electronics package 14 may be skull mounted for a cortical visual prosthesis, cochlear stimulator or deep brain stimulator. The electronics package 14 would be preferably mounted on the back of the head for a cortical visual prosthesis, the top of the head for a deep brain stimulator, or the side of the head for a cochlear prosthesis. When the electronics package 14 is mounted on the skull there may be a need to make the electronics package 14 more impact resistant. For example, the case might be designed to withstand impacts of 100 pounds per square inch or even greater forces up to 400 pounds per square inch. **FIGS. 25A** and **25B** present the electronics package 14 with an extended wall 302. The extended wall 302 extends beyond the array cable 12 and lead to coil 16. Silicone fill 300 fills the volume inside the extended wall 302 providing further impact protection of the ceramic substrate 60. The extended wall 302 defines a first slot 304 for the array cable 12 and a second slot 306 for the lead to coil 16. Hence any impact to the electronics package 14 will be transferred to the skull underlying the extended wall 302 rather than to the array cable 12 or lead to coil 16. Silicone fill 300 will locate the array cable 12 and lead to the coil 16 within the slots 304 and 306 to further protect the array cable 12 and lead to the coil 16. In a further alternate embodiment shown in **FIGS. 26A** and **26B** the extended wall 302 is provided with a flange 308 to distribute any impact applied to electronics package 14 over a larger area of the skull reducing the likelihood of a skull fracture resulting from an impact. This flange can extend beyond the extent of the can (as shown) or alternately may face inwards to reduce the horizontal extent of the can. In a still further embodiment, presented in **FIGS. 27A** and **27B**, a metal base 310 is bonded to the flange 308 to further protect the electronics package 14 and ceramic substrate. This can be done with an inward flange (not shown) or an outward flange 308 as shown. The base 310 is bonded by various methods such as adhesives, but is preferably laser welded at the edges. In this embodiment, silicone 300 again fills the space between the ceramic substrate 60 and the metal base. The metal base is preferably made from the same material as the metal flange 308 and extended wall 302. Examples include titanium CP, titanium 6-4, niobium, etc.

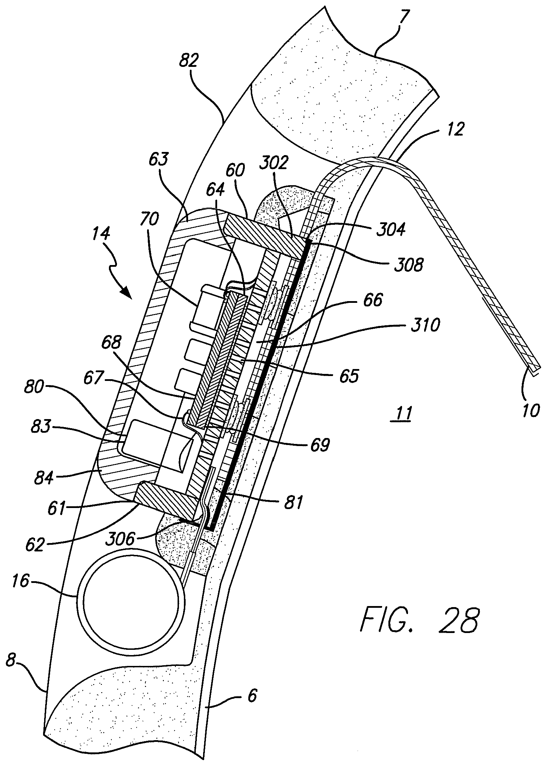

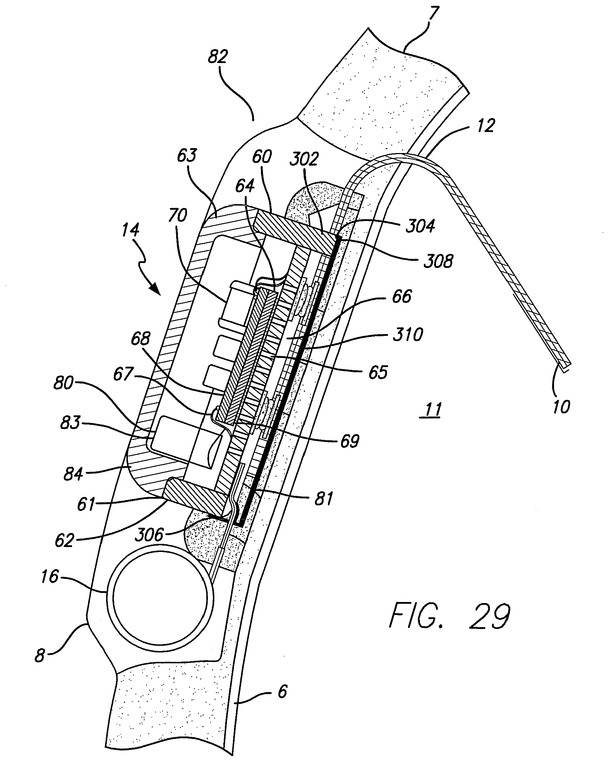

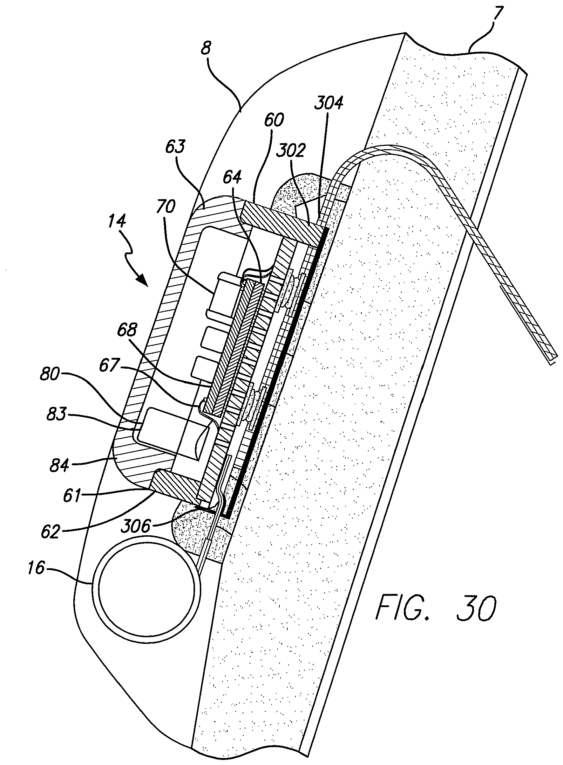

**FIG. 28** shows the preferred placement of the electronics package 14. The electronics package 14 is attached to the cranium 7. Alternatively, the package 14 may be affixed to the cranium through the use of one or more straps, or the package 14 may be glued to the cranium using a reversible or non-reversible adhesive attach. In this embodiment the package, which is imbedded within the cranium, is low profile and shaped in manner that permits the scalp 8 to rest on top of the package with little or no irritation of the scalp. Additionally, edges of the package are preferably rounded and or the package 14 may be encased in a soft polymer mold such as silicone to further reduce irritation. In other embodiments the package 14 may be attached to the scalp 8, brain 11 or dura 12. The electrode array 10 as shows is shaped for a deep brain stimulator. As noted above, the invention is useful for a cortical visual prosthesis or cochlear prosthesis with changes to the electrode array 10 and array cable 12. As shown, the extended wall 302, flange 304 and base 310 rest on the skull 7 to provide the maximum impact protection. If a larger package is need, as presented in **FIG. 29**, the package may extend beyond the skull 7 with the scalp 8 extending over the package 14. Silicone or other soft material packed around the electronics package 14 to provide a smooth shape to the scalp. **FIG. 30** presents a further embodiment with the impact resistant package on the surface of the skull 7 under the scalp 8. It is sometimes necessary to mount the package on the surface of the skull, when, for example, hollowing out the bone of the skull is not practical, such as in a child where the bone is precariously thin. Also if the package is small enough, hollowing out bone is not necessary.

Accordingly, what has been shown is an improved method making a hermetic package for implantation in a body. While the invention has been described by means of specific embodiments and applications thereof, it is understood that numerous modifications and variations could be made thereto by those skilled in the art without departing from the scope of the invention. It is therefore to be understood that within the scope of the claims, the invention may be practiced otherwise than as specifically described herein.

## Claims

1. An implantable device comprising:
a hermetic package enclosing electronics, the hermetic package having a first set of contact pads (222) on its surface;
a flexible circuit (218) including a second set of contact pads (232) on its surface aligned with said first set of contact pads;
a roughened surface having a surface area greater than five times that of a shiny platinum surface of the same geometry as that of the roughened surface on at least one contact pad of at least one of said first set of contact pads or said second sets of contact pads; and
conductive adhesive (281) between said first set of contact pads and said second set of contact pads.

2. The implantable device according to claim 1, further comprising a nonconductive adhesive underfill between said hermetic package and said flexible circuit around said conductive adhesive.

3. The implantable device according to claim 1, wherein said roughened surface comprises an electroplated surface.

4. The implantable device according to claim 3, wherein said electroplated surface comprises platinum gray.

5. The implantable device according to claim 1, wherein said roughened surface comprises a sputtered surface or a surface applied by chemical vapor deposition.

6. The implantable device according to claim 1, wherein said roughened surface comprises an etched surface.

7. The implantable device according to claim 6, wherein said etched surface is etched by reactive ion etching; etched by a laser; or etched by sandblasting.

8. A method of making an implantable device comprising:
providing a hermetic package enclosing electronics, the hematic package having a first set of contact pads on its surface;
providing a flexible circuit including a second set of contact pads on its surface aligned with said first set of contact pads;
roughening the surfaces on at least a portion of said first set of contact or said second set of contact pads to give a surface area greater than five times that of a shiny platinum surface of the same geometry as that of the roughened surface; and
bonding said first set of contact pads with said second set of contact pads using conductive adhesive.

9. The method according to claim 8, further comprising applying a nonconductive adhesive underfill between said hermetic package and said flexible circuit and around said conductive adhesive.

10. The method according to claim 8, wherein said step of roughening said surface is electroplating.

11. The method according to claim 10, wherein said electroplating is electroplating with platinum gray.

12. The method according to claim 8, wherein said step of roughening is sputtering or a chemical vapor deposition.

13. The method according to claim 8, wherein said step of roughening is etching.

14. The method according to claim 13, wherein said etching is etching by reactive ion etching, etching by a laser, or by sandblasting.

## Patentansprüche

1. Implantierbare Vorrichtung, umfassend:
ein Elektronik umschließendes, hermetisches Gehäuse, wobei das hermetische Gehäuse eine erste Gruppe von Kontaktstellen (222) auf seiner Oberfläche aufweist;
eine flexible Schaltung (218), die eine zweite Gruppe von Kontaktstellen (232) auf ihrer Oberfläche umfasst, die mit der ersten Gruppe von Kontaktstellen fluchtend ausgerichtet ist;
eine aufgeraute Oberfläche, die einen Oberflächenbereich aufweist, der fünf Mal größer als derjenige einer glänzenden Platinoberfläche derselben Geometrie wie diejenige der aufgerauten Oberfläche auf mindestens einer Kontaktstelle der ersten Gruppe von Kontaktstellen oder der zweiten Gruppe von Kontaktstellen ist; und
einen leitfähigen Klebstoff (281) zwischen der ersten Gruppe von Kontaktstellen und der zweiten Gruppe von Kontaktstellen.

2. Implantierbare Vorrichtung gemäß Anspruch 1, ferner umfassend eine nichtleitende Klebstoff-Unterfüllung zwischen dem hermetischen Gehäuse und der flexiblen Schaltung um den leitfähigen Klebstoff herum.

3. Implantierbare Vorrichtung gemäß Anspruch 1, worin die aufgeraute Oberfläche eine galvanisierte Oberfläche umfasst.

4. Implantierbare Vorrichtung gemäß Anspruch 3, worin die galvanisierte Oberfläche Platingrau umfasst.

5. Implantierbare Vorrichtung gemäß Anspruch 1, worin die aufgeraute Oberfläche eine gesputterte Oberfläche oder eine durch chemische Gasphasenabscheidung aufgetragene Oberfläche umfasst.

6. Implantierbare Vorrichtung gemäß Anspruch 1, worin die aufgeraute Oberfläche eine geätzte Oberfläche umfasst.

7. Implantierbare Oberfläche gemäß Anspruch 6, worin die geätzte Oberfläche durch reaktives lonenätzen geätzt wird; durch einen Laser geätzt wird; oder durch Sandstrahlen geätzt wird.

8. Verfahren zur Herstellung einer implantierbaren Vorrichtung, umfassend: Bereitstellen eines Elektronik umschließenden, hermetischen Gehäuses, wobei das hermetische Gehäuse eine erste Gruppe von Kontaktstellen auf seiner Oberfläche aufweist;
Bereitstellen einer flexiblen Schaltung, die eine zweite Gruppe von Kontaktstellen auf ihrer Oberfläche umfasst, die mit der ersten Gruppe von Kontaktstellen fluchtend ausgerichtet ist;
Aufrauen der Oberflächen auf mindestens einem Abschnitt der ersten Gruppe von Kontaktstellen oder der zweiten Gruppe von Kontaktstellen, um einen Oberflächenbereich zu erzeugen, der fünf Mal größer als derjenige einer glänzenden Platinoberfläche derselben Geometrie wie diejenige der aufgerauten Oberfläche ist; und
haftschlüssiges Verbinden der ersten Gruppe von Kontaktstellen mit der zweiten Gruppe von Kontaktstellen durch Verwendung eines leitfähigen Klebstoffs.

9. Verfahren gemäß Anspruch 8, ferner umfassend das Auftragen einer nichtleitenden Klebstoff-Unterfüllung zwischen dem hermetischen Gehäuse und der flexiblen Schaltung und um den leitfähigen Klebstoff herum.

10. Verfahren gemäß Anspruch 8, worin der Schritt des Aufrauens der Oberfläche ein Galvanisieren ist.

11. Verfahren gemäß Anspruch 10, worin das Galvanisieren ein Galvanisieren mit Platingrau ist.

12. Verfahren gemäß Anspruch 8, worin der Schritt des Aufrauens ein Sputtern oder eine chemische Gasphasenabscheidung ist.

13. Verfahren gemäß Anspruch 8, worin der Schritt des Aufrauens ein Ätzen ist.

14. Verfahren gemäß Anspruch 13, worin das Ätzen ein Ätzen durch reaktives lonenätzen, Ätzen durch einen Laser oder durch Sandstrahlen ist.

## Revendications

1. Dispositif implantable comprenant :
un boîtier hermétique enfermant une électronique, le boîtier hermétique comportant un premier ensemble de pastilles de contact (222) sur sa surface ;
un circuit souple (218) comprenant un deuxième ensemble de pastilles de contact (232) sur sa surface aligné avec ledit premier ensemble de pastilles de contact ;
une surface rugosifiée ayant une aire de surface cinq fois plus grande que celle d'une surface de platine brillante avec la même géométrie que celle de la surface rugosifiée sur au moins une pastille de contact d'au moins l'un dudit premier ensemble de pastilles de contact ou dudit deuxième ensemble de pastilles de contact ; et
un adhésif conducteur (281) entre ledit premier ensemble de pastilles de contact et ledit deuxième ensemble de pastilles de contact.

2. Dispositif implantable selon la revendication 1, comprenant en outre une sous-épaisseur adhésive non conductrice entre ledit boîtier hermétique et ledit circuit souple autour dudit adhésif conducteur.

3. Dispositif implantable selon la revendication 1, dans lequel ladite surface rugosifiée comprend une surface électroplaquée.

4. Dispositif implantable selon la revendication 3, dans lequel ladite surface électroplaquée comprend du gris platine.

5. Dispositif implantable selon la revendication 1, dans lequel ladite surface rugosifiée comprend une surface pulvérisée ou une surface appliquée par dépôt chimique en phase vapeur.

6. Dispositif implantable selon la revendication 1, dans lequel ladite surface rugosifiée comprend une surface gravée.

7. Dispositif implantable selon la revendication 6, dans lequel ladite surface gravée est gravée par gravure ionique réactive ; gravée par laser ; ou gravée par sablage.

8. Procédé de fabrication d'un dispositif implantable comprenant :
la fourniture d'un boîtier hermétique enfermant une électronique, le boîtier hermétique comportant un premier ensemble de pastilles de contact sur sa surface ;
la fourniture d'un circuit souple comprenant un deuxième ensemble de pastilles de contact sur sa surface aligné avec ledit premier ensemble de pastilles de contact ;
la rugosification des surfaces sur au moins une partie dudit premier ensemble de pastilles de contact ou dudit deuxième ensemble de pastilles de contact pour obtenir une aire de surface cinq fois plus grande que celle d'une surface de platine brillante avec la même géométrie que celle de la surface rugosifiée ; et
la liaison dudit premier ensemble de pastilles de contact avec ledit deuxième ensemble de pastilles de contact en utilisant un adhésif conducteur.

9. Procédé selon la revendication 8, comprenant en outre l'application d'une sous-épaisseur adhésive non conductrice entre ledit boîtier hermétique et ledit circuit souple et autour dudit adhésif conducteur.

10. Procédé selon la revendication 8, dans lequel ladite étape de rugosification de ladite surface est un dépôt électrolytique.

11. Procédé selon la revendication 10, dans lequel ledit dépôt électrolytique est un dépôt électrolytique avec du gris platine.

12. Procédé selon la revendication 8, dans lequel ladite étape de rugosification est une pulvérisation ou un dépôt chimique en phase vapeur.

13. Procédé selon la revendication 8, dans lequel ladite étape de rugosification est une gravure.

14. Procédé selon la revendication 13, dans lequel ladite gravure est une gravure par gravure ionique réactive, par gravure au laser, ou par sablage.
